# EUROPEAN PATENT SPECIFICATION

(11) **EP 4 021 566 B1**
(45) Date of publication and mention of the grant of the patent: **14.05.2025**
(21) Application number: 20859164.4
(22) Date of filing: 26.08.2020
(51) Int. Cl.: A61N 1/39

(54) **AUTOMATED WEARABLE BELT CARDIAC DEFIBRILLATOR**
AUTOMATISIERTER, AN EINEM GÜRTEL TRAGBARER HERZDEFIBRILLATOR
DÉFIBRILLATEUR CARDIAQUE AUTOMATISÉ VESTIMENTAIRE SE PORTANT COMME UNE CEINTURE

(30) Priority: 27.08.2019 US 201962892090 P
(43) Date of publication of application: 06.07.2022
(73) Proprietor: Newpace Ltd., 3088900 Caesarea (IL)
(72) Inventor: STROMMER, Gera, 3498129 Haifa (IL); BRODER, Avraham, 4971202 Petach Tikva (IL); FISHEL, Robert, Delray Beach, Florida 33483 (US); SHMARAK, Itzik, 3600100 Nofit (IL)
(74) Representative: Kasche & Partner
(86) International application number: PCT/IB2020/057988
(87) International publication number: WO 2021/038471

(56) References cited:
- WO-A1-2017/035502
- WO-A2-2015/127466
- US-A- 5 348 008
- US-A1- 2002 072 682
- US-A1- 2012 112 903
- US-A1- 2016 136 466
- US-A1- 2017 007 129
- US-A1- 2017 143 977
- US-A1- 2017 143 977
- US-A1- 2018 140 859
- US-A1- 2018 169 426
- US-A1- 2018 272 145
- US-A1- 2018 272 145
- US-A1- 2019 022 400

## Description

### CROSS REFERENCE TO RELATED APPLICATIONS

This application claims the benefit of U.S. Provisional patent application No. 62/892,090 filed August 27, 2019.

### FIELD

Embodiments disclosed herein relate to systems and methods for a wearable lifesaving defibrillator.

### BACKGROUND

Automated External Defibrillators (AED) are portable devices used to defibrillate patients having a sudden cardiac arrest (SCD) event, generally due to ventricular tachycardia (VT) or ventricular fibrillation (VF). When a person loses consciousness, and there is a chance of VT or VF, an AED is brought to the patient for use. An AED generally functions as follows: two patches are affixed to the subject's chest and abdomen; the AED detects the heart rate and analyses the subject's heart condition using ECG electrodes integrated into the patches; and, if VF or VT are detected, the AED automatically delivers a high voltage and high energy electrical shock to the subject through the patches. This process differs from a standard defibrillator in which the high energy, high voltage shock is initiated by personnel operating the defibrillator. With an AED, the process of analyzing the heart condition and delivering of the shock is automated. The AED patches are adhered to the patient using a short-term adhesive since the patches are generally attached for not more than one hour. Further, since the patient has lost consciousness and is lying horizontally, the patches and adhesive are not designed for vertical adhesion or movement of the patient or for carrying the patch's weight.

Wearable Cardiac Defibrillators (WCD) differ from regular AEDs as WCDs are worn continuously by patients who are at high risk of sudden cardiac arrest (SCA) caused by VT or VF to provide continuous protection to the patient. Unless the VF is detected and defibrillation administered within a few seconds, VF and related SCA may cause sudden cardiac death (SCD). Patients who are at high risk for VF are candidates for an implantable cardioverter defibrillator (ICD). During the waiting period until the ICD implantation (in case such an implantation is not immediate), or in cases where the indication for ICD implantation is not sufficiently clear, a WCD is often used. The functionality provided by a WCD is thus similar to that of an ICD, but provided for a limited time period and without the need for an invasive implanting procedure.

WCDs usually include two types of patches/electrodes: sensing electrodes or patches and shocking electrode or patches. The sensing electrodes or patches are similar to standard ECG patches in function and are used to sense the ECG of the patient in order to detect VF. The defibrillation electrodes or patches are used to transmit a high voltage and high energy defibrillation shock to the patient's heart in a case of SCA. These shocks usually have a voltage in the range of 1000V-3000V and energy in the range of 150-300 Joules. In practice the voltage and energy to be delivered depends on the patient impedance, which is usually measured prior to shock delivery.

Some current WCDs consist of sensing patches and shocking electrode patches that are stuck onto the patient's skin using some form of adhesive. Since a wearable defibrillator must be used on a 24/7 basis and often for extended periods of time (several days and typically over one week), patches that are in contact with the patient's body via the adhesive may cause discomfort for the patient and skin irritations in some cases, and make it difficult to perform normal daily activities. The discomfort from the adhesive patches is therefore a function of both the time used and also the amount of activity.

To resolve the skin discomfort from adhesives, other current WCDs consist of a wearable vest/top where the sensing patches and shocking electrode patches are pressed against the patient's skin, usually by one or more tightening straps such as over-shoulder and torso straps. In order to sufficiently deliver the high energy defibrillation pulse, a good connection is required between the shocking electrode patches and the patient's skin. Since the patches in the vest are not stuck to the patient's body, they are held in tight direct contact with the patient's skin by the vest and also feature a gel release mechanism to enhance skin contact required during shock delivery. Tight vests force electrodes into contact with the patient's body, thus also causing major discomfort and making it difficult to perform normal daily activities. Additionally, the vest itself is in direct contact with the patient's skin and can also cause significant discomfort. Further the vest needs to be washed on a daily basis while active elements are removed and repositioned in a secondary vest, forcing the patient to ensure the repositioned active elements are placed correctly on a daily basis. In some known cases patients were not protected since the active elements were incorrectly positioned by the patient. Finally, such daily maintenance activity may not be appropriate for elderly patients or patients who need care or assistance.

Further, the defibrillation electrical elements (batteries, capacitors and electronic circuits) are usually provided in a box that must be worn, such as with a strap or clamp onto clothing, resulting in a cumbersome source of discomfort to the patient. These causes of discomfort (patches, vest, box) may result in the patient removing the vest or patches, thus causing compliance issues and potentially endangering the life of the patient.

Finally, although wearable defibrillators feature ECG patches attached to a patient, they generally do not make use of these for integration with a cardiac telemetry system. Cardiac telemetry is usually provided by devices such as Holter monitors or implantable recorder systems that feature ongoing heart monitoring.

US20170056682A1 discloses a wearable external defibrillator with a plurality of ECG sensing electrodes and a first defibrillator pad electrode and a second defibrillator pad electrode. The ECG sensing electrodes and the defibrillator pad electrodes are configured for long term wear.

### SUMMARY

Exemplary embodiments disclosed herein relate to a wearable automated Belt Cardiac Defibrillator (BCD).

The BCD as disclosed herein is adapted so as provide AED functionality and automated ease of use in a wearable form that is comfortable for long term use by a subject. Improvements in comfort are provided by several innovations as described further herein:
- Combined replaceable, light, and flexible defibrillation and sensor patches are attached using a biocompatible adhesive. The patches and adhesive are designed for long term adhesion to a patient enabling the patient to continue normal daily activities;
- The combined patches can be repositioned periodically to reduce skin irritation. Multiple different defibrillation and sensing vectors corresponding to several different patch positions are proposed such that the patches positions can be altered;
- The defibrillator controller and electrical elements are packaged into a flexible, slim, low profile and lightweight wearable belt. The belt can be worn in multiple positions or carried, and the defibrillator controller and electrical elements are stored in several interconnected compartments providing flexibility to the belt while keeping the belt lightweight and easy to wear.

In some other embodiments, a proposed BCD includes defibrillation electrodes and ECG sensors mounted in a flexible removable patch strip for adhesion to the patient or insertion into a stretchable, washable top.

Further, in some embodiments, the BCD as disclosed performs cardiac telemetry by continuously monitoring and analyzing the subject's heart using the ECG sensor and controller, and reporting detected arrhythmias to a patient's phone and/or to a remote monitoring center.

In some embodiments, a flexible automated wearable belt cardiac defibrillator (BCD) for wearing by a subject, comprises: at least two patches adapted for adhering to the subject each comprises a defibrillation electrode and at least one ECG sensor; and a BCD controller connected to each of the patches, wherein the patches comprise an adhesive adapted for long-term adhering of the patches to the subject, wherein the patches and adhesive are adapted for movement of the subject while the patches are adhered to the subject, wherein the patches are replaceable, wherein the controller is housed in a flexible belt comprises a plurality of compartments for wearing by the subject, wherein the controller is adapted for being flexible, and wherein the adaptation for being flexible comprises distributing the controller components between the plurality of compartments.

In some embodiments, the controller components comprise a battery, at least one high voltage and high energy capacitor, and controller electronics, and each of the controller components is housed in a separate one of the plurality of compartments. In some embodiments, the belt comprises a flexible material. In some embodiments, "long-term" is between one day and 10 days. In some embodiments, "long-term" may be even longer than 10 days. In some embodiments, the compartments have the form of pockets. In some embodiments, the patches comprise a gel for enhancing electrical contact. In some embodiments, the adhesive is a biocompatible adhesive.

In some embodiments, at least one of the patches comprises a vibration element. In some embodiments, the vibration element is adapted for vibrating to alert the subject prior to an impending shock delivery by the defibrillation electrode. In some embodiments, the weight of the controller components is distributed around the belt. In some embodiments, the patches are small patches having dimensions of less than 8x8cm. In some embodiments, at least three patches for providing at least two defibrillation vectors. In some embodiments, the controller comprises a controller vibration pad.

In some embodiments, the controller vibration pad is adapted to generate vibration to alert the subject of an impending shock to be delivered by the defibrillation electrode. In some embodiments, the controller comprises an audio output device adapted to generate an alarm sound in case of an impending a shock about to be delivered by the defibrillation electrode. In some embodiments, the audio output device is adapted to sound an alarm following administering of a defibrillating shock. In some embodiments, the controller comprises a microphone adapted for recording the audio output of the subject.

In some embodiments, the controller is adapted for storage and/or analysis of ECG sensor data sensed by the ECG sensor. In some embodiments, the controller comprises an ECG event button and pressing the event button creates a time stamp in the collected ECG sensor data. In some embodiments, pressing of the ECG event button initiates recording via the microphone. In some embodiments, the controller is adapted to measure impedance between the patches and the body of the subject to determine the strength of the electrical connection therebetween. In some embodiments, the controller comprises an abort button adapted for aborting an impending shock from the defibrillation electrode. In some embodiments, the BCD further comprises a safety switch and the safety switch is adapted to electrically disconnect the controller from the defibrillation electrode.

In some embodiments, the controller comprises a controller motion sensor and/or the BCD comprises an ECG sensor motion sensor. In some embodiments, one or both of controller motion sensor and the ECG motion sensor are adapted for determining data related to movement of the subject. In some embodiments, the patches comprise multiple perforations throughout the patches. In some embodiments, the patches comprise connectors for connecting or disconnecting interconnection cables between the patches and/or between the patches and the controller.

In some embodiments, the controller is adapted for data communication with a remote monitoring center (RMC). In some embodiments, ECG data sensed by the ECG sensor is transmitted to the RMC for analysis and storage. In some embodiments, detected arrhythmia events are transmitted to the RMC. In some embodiments, the controller comprises a GPS receiver adapted for determining the geolocation of the BCD, wherein the determined location is transmitted to the RMC. In some embodiments, the controller comprises a microphone and an audio device and is adapted for initiating a voice call with the RMC.

In some embodiments, the controller is adapted for wireless communication with an external device. In some embodiments, the external device presents the status of the BCD using a display of the external device. In some embodiments, audible, visual and/or vibrating indication of an impending shock to be delivered by the defibrillator electrode is provided by the external device using display, audio, and/or vibrating functions of the external device. In some embodiments, the external device comprises an abort button for aborting an impending shock to be delivered by the defibrillator electrode. In some embodiments, the BCD is adapted to deliver high voltage temporary external percutaneous heart pacing via the patches following detection by the BCD of asystole after delivering a shock. In some embodiments, the pacing is ventricular demand pacing. In some embodiments, the pacing is provided at a rate of 35 BPM.

In some embodiments, the BCD functions without the need for any other part of the BCD to be in contact with the subject's skin other than the patches. In some embodiments, the controller is not attached to the subject via a patch touching the subject skin. In some embodiments, the patches include at least two ECG sensor electrodes for ECG sensing from at least one sensing vector. In some embodiments, the patches include electrodes delivering a high voltage and high energy defibrillation shock from at least one shocking vector.

In some embodiments, a method for usage of an BCD comprises: providing the BCD as described above; adhering of the patches to the subject; operating the BCD; and following completion of an operational period, positioning of the patches to alternative locations on the subject wherein each of the alternate locations represents an alternate shock vector.

In some embodiments, the alternate shock vector is selected from the list consisting of: a first shock vector comprises a first patch on a right side of the chest and a second patch on a left side of the abdomen, a second shock vector comprises the first patch on a left side of the chest and the second patch on a right side of the abdomen and a third shock vector comprises the first patch on an upper right side of the chest and the second patch on an upper left side of the back.

In some embodiments, the controller is adapted to indicate completion of the operational period for movement of the patches. In some embodiments, the external device is adapted to provide guided instructions for positioning of the patches.

In some embodiments, a BCD for wearing by a subject, comprises: a removable patch strip (RPS) comprising at least one defibrillation patch and at least one ECG sensor; and a top comprising a pocket for insertion of the RPS, such that the at least one defibrillation patch and the at least one ECG sensor are pressed against the torso of the subject. In some embodiments, the top comprises a stretchable material. In some embodiments, the top comprises a washable material.

As used herein, the terms "patch" or "pad" describe a patch incorporating one or both of a sensing electrode and a defibrillation electrode. The terms "patient" and "subject" are used herein interchangeably.

Unless otherwise defined, all technical and scientific terms used herein have the same meaning as commonly understood by one of ordinary skill in the art. The materials, methods, and examples provided herein are illustrative only and not intended to be limiting.

Implementation of the method and system of the present disclosure may involve performing or completing certain selected tasks or steps manually, automatically, or a combination thereof. Moreover, according to actual instrumentation and equipment of preferred embodiments of the method and system of the present disclosure, several selected steps may be implemented by hardware (HW) or by software (SW) on any operating system of any firmware, or by a combination thereof. For example, as hardware, selected steps of the disclosure could be implemented as a chip or a circuit. As software or algorithm, selected steps of the disclosure could be implemented as a plurality of software instructions being executed by a computer using any suitable operating system. In any case, selected steps of the method and system of the disclosure could be described as being performed by a data processor, such as a computing platform for executing a plurality of instructions.

Although the present disclosure is described with regard to a "computing device", a "computer", or "mobile device", it should be noted that optionally any device featuring a data processor and the ability to execute one or more instructions may be described as a computer, including but not limited to any type of personal computer (PC), a server, a distributed server, a virtual server, a cloud computing platform, a cellular telephone, an IP telephone, a smartphone, a smart watch or a PDA (personal digital assistant). Any two or more of such devices in communication with each other may optionally comprise a "network" or a "computer network".

### BRIEF DESCRIPTION OF THE DRAWINGS

Aspects, embodiments and features disclosed herein will become apparent from the following detailed description when considered in conjunction with the accompanying drawings. Like elements may be marked with like numerals in different figures, where:
FIG. 1 shows an exemplary schematic drawing of a BCD according to some embodiments;
FIGS. 2A-2D show exemplary schematic drawings of a BCD according to some embodiments;
FIG. 3 shows an exemplary schematic drawing of a BCD according to some embodiments;
FIG. 4 shows an exemplary schematic drawing of a BCD according to some embodiments;
FIGS. 5A-5G show exemplary schematic drawings of a BCD according to some embodiments;
FIGS. 6A-6B show exemplary schematic drawings of a BCD according to some embodiments;
FIGS. 7A-7B show exemplary schematic drawings of the components of a BCD according to some embodiments.

### DETAILED DESCRIPTION

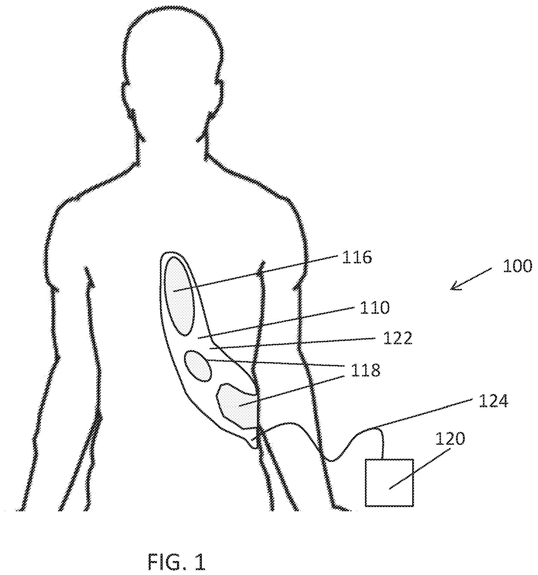

Exemplary embodiments disclosed herein relate to a wearable AED (BCD) and method of use. FIG. 1 shows an exemplary schematic drawing of a BCD according to some embodiments. FIG. 1 shows a BCD 100 comprising a removable patch strip (RPS) 110 and a defibrillator controller 120. RPS 110 comprises one or more defibrillation (shocking) patches 116 and ECG sensors 118. RPS 110 comprises a soft structural base 122 comprising a soft material. In some embodiments, RPS 110 is biocompatible. In some embodiments, RPS 110 is held in place on the subject using a biocompatible adhesive (not shown). Defibrillator controller 120 monitors the subject's heart using sensors 118 and triggers defibrillation patches 116 if required. Sensors 118 and defibrillation patches 116 are connected to defibrillator controller 120 using wires 124. In some embodiments, defibrillation patches 116 comprise either a constant gel layer or a gel release mechanism (not shown) for releasing gel prior to defibrillation to improve conductivity of the defibrillation shock.

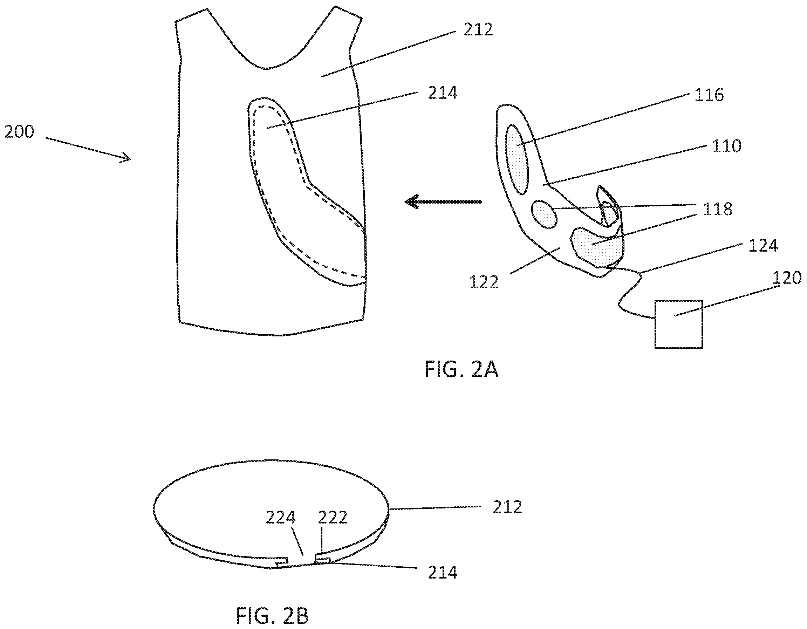

FIGS. 2A-2D show exemplary schematic drawings of a BCD according to some embodiments. As shown in FIGS. 2A-2D, a BCD 200 comprises a stretchable top 212, an RPS 110 and a controller 120. RPS 110 comprises one or more defibrillation (shocking) patches 116 and ECG sensors 118. RPS 110 is sized to removably slide into a pocket 214 of top 212. In some embodiments, top 212 comprises a stretch material. In some embodiments, top 212 comprises a washable material.

As shown in the plan view of FIG. 2B, pocket 214 has borders 222 made to hold RPS 110 firmly in position while leaving an opening 224 for RPS 110 to touch the torso of the subject. ECG sensors 118 and defibrillation patches 116 are therefore continuously in contact with the skin.

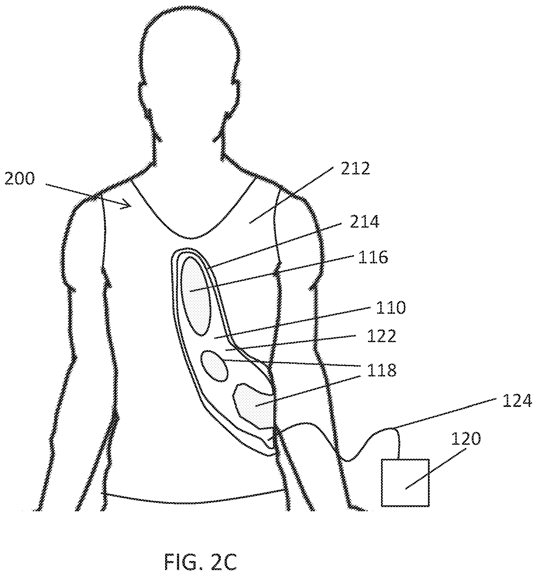

FIG. 2C shows RPS 110 inserted into pocket 214. It should be appreciated that stretchable top 212 holds RPS 110 and thus sensors 118 and defibrillation patches 116 pressed against the torso of a subject without the need for adhesives or adjusting straps. Further, a top 212 can be switched with another top 212 where RPS 110 can be removed from a first top 212 and placed in a second top 212. This exchange can allow for laundering of the first top 212, and vice versa.

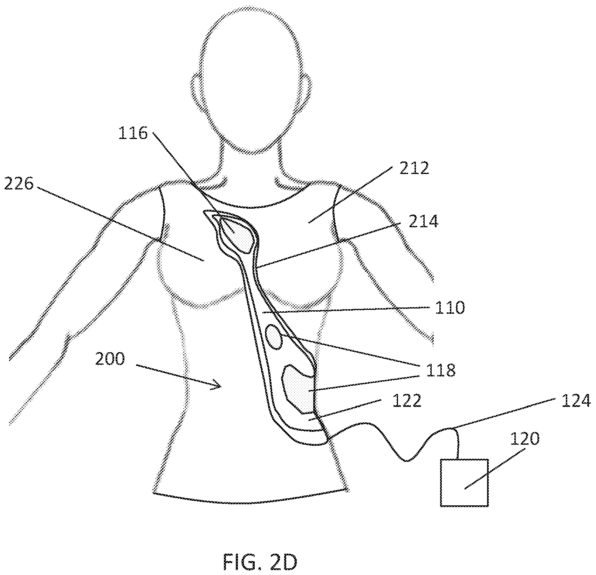

In some embodiments, BCD 200 is adapted for use by a female subject. As shown in FIG. 2D, RPS 110 and pocket 214 are adapted to position defibrillation patches 116 above the right breast 226 of the subject.

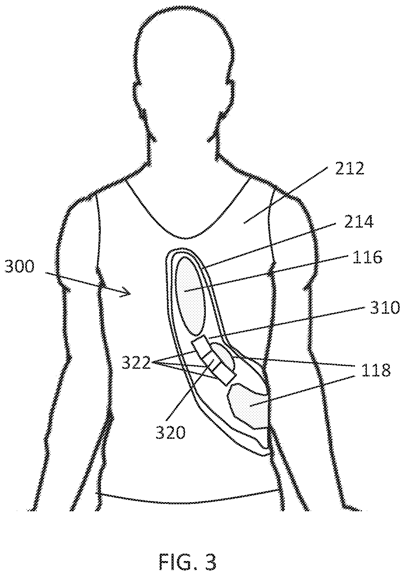

FIG. 3 shows an exemplary schematic drawing of a BCD according to some embodiments. As shown in FIG. 3, a BCD 300 comprises a top 212, an RPS 310 and a defibrillator controller 320. BCD 300 is the same as BCD 200 apart from defibrillator controller 220 which is replaced with defibrillator controller 320. Controller 320 is integrated into RPS 310 and the components of controller 320 are distributed across RPS 310. This distribution spreads the controller components over a larger area (compared to controller 220) and thus provides enhanced weight distribution and greater comfort for the patient. In some embodiments, a housing 322 of controller 320 is cylindrical. In some embodiments, multiple housings 322 are provided for the components of controller 320.

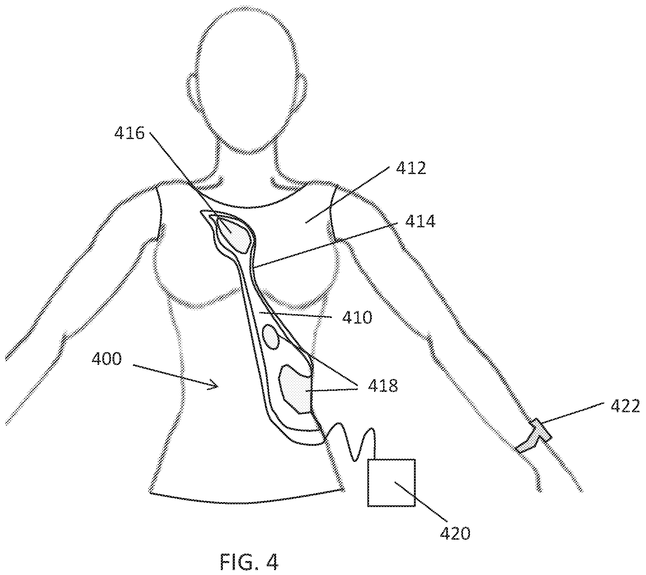

FIG. 4 shows an exemplary schematic drawing of a BCD according to some embodiments. As shown in FIG. 4, a BCD 400 comprises a stretchable top 412, an RPS 410, a defibrillator controller 420 and an external device 422. RPS 410 comprises one or more defibrillation patches 416 and ECG sensors 418. RPS 410 is sized to removably slide into a pocket 414 of a top 412 of BCD 400. Top 412 is the same as top 212 described above.

Non-limiting examples of external device 422 are a smart watch or smartphone. External device 422 is in wireless communication with defibrillation patches 416, ECG sensors 418 and defibrillation controller 420. A non-limiting example of a wireless communication protocol is Bluetooth. External device 422 is a computing device and runs software to monitor the subject's heart using data received wirelessly from sensors 418, and triggers defibrillation patches 416 if required by communicating with controller 420 and/or defibrillation patches 416. In some embodiments, controller 420 is integrated into RPS 410 similarly to the way controller 320 is integrated into RPS 310.

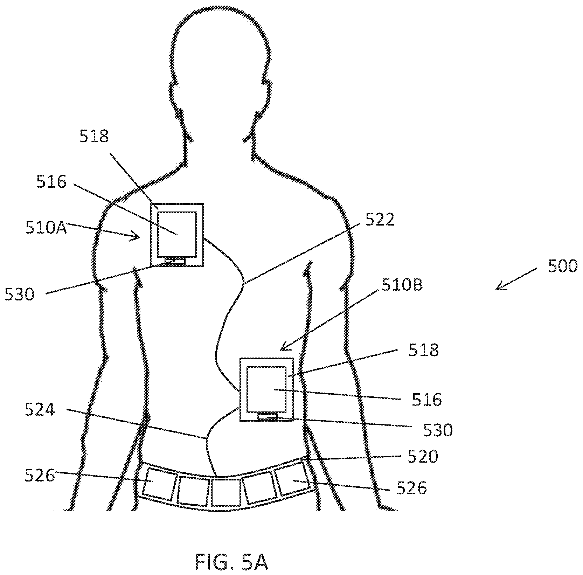

FIGS. 5A-5G show exemplary schematic drawings of a BCD according to some embodiments. As shown in FIG. 5A BCD 500 comprises replaceable patches 510A and 510B (referred to also simply as "patches 510") and a defibrillation controller belt (or simply "belt") 520. Each of patches 510A, 510B comprises both a defibrillation electrode 516 and an ECG sensor 518.

In some embodiments, each of patches 510 includes include three or more ECG sensors 518 for sensing simultaneous sensing vectors. Multiple sensing electrodes per EC sensor 518 provides better sensing and detection of VF and discrimination of non-VF arrhythmias.

In some embodiments, each of patches 510 includes three or more defibrillation electrodes 516 for providing multiple simultaneous shock vectors. for better coverage of the heart and reduction of defibrillation threshold energy.

Patches 510A, 510B are affixed to the body of the subject using a biocompatible adhesive that is non-irritating for skin, is adapted both for extended use and for ensuring adhesion while that the subject continues to be active and mobile. A non-limiting example of a suitable adhesive may be KM40C by Katecho LLC of Des Moines, Iowa. In some embodiments, extended use covers a period of over 7 days. In some embodiments, extended use covers a period of over 10 days.

In some embodiments, patches 510A, 510B comprise gel for enhancing electrical contact. In some embodiments, defibrillation electrode 516 performs ECG signal measurement and ECG sensor 518 is not required.

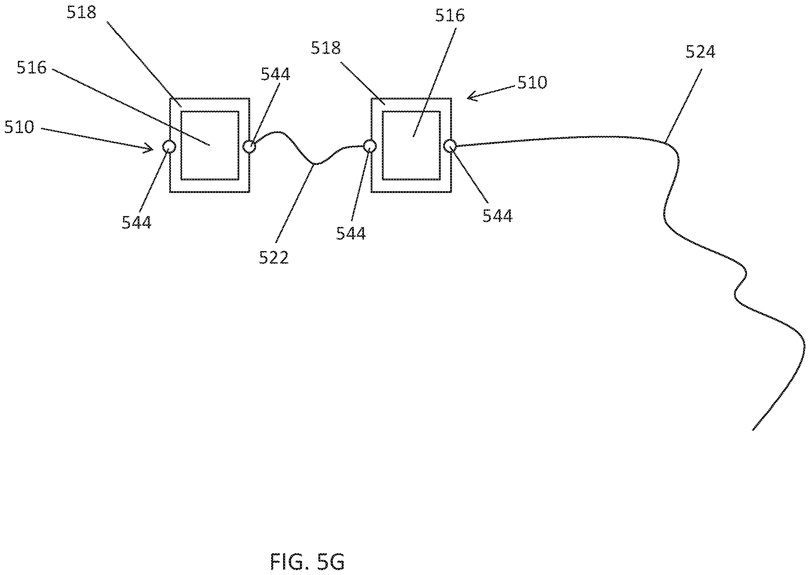

In some embodiments, replaceable patches 510A, 510B include a vibration element 530 used to alert the patient prior to shock delivery. In some embodiments, replaceable patches 510A, 510B are replaced periodically. In some embodiments, replaceable patches 510A, 510B are disposable. In some embodiments, BCD 500 comprises more than two patches 510. Cables 522 provide electrical connectivity between patches 510 and cables 524 provide electrical connectivity between patches 510 and controller belt 520. As shown in FIG. 5G, each of patches 510 includes connectors 544 for connecting of cables 522 and 524 to patches 510. When one or more of patches 510 are replaced, they are disconnected at connectors 544 from cables 522 and/or 524, and new patches 510 also including connectors 544 are connected to cables 522 and/or 524. In some embodiments, controller belt 520 includes a switching component in order to carry both of sensing signals and shock current on the same electrode.

Controller belt 520 has the form of a flexible belt. Belt 528 comprises a flexible material. Controller belt 520 is flexible and wearable and comprises an adjustment means 542 such a belt adjustment means known in the art in order to fit controller belt 520 comfortably around the waist or torso of the subject. Alternatively, belt 520 may be worn on another part of the body or may be carried.

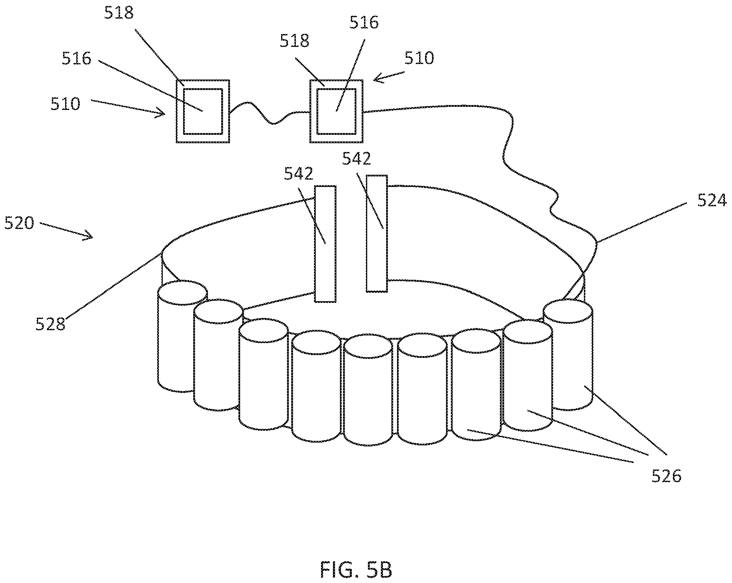

Controller belt 520 contains all the components of a defibrillator controller (such as controller 720, high voltage capacitors, batteries, electronics, high voltage shock circuits, alarm system, communication system, operating software as well as detection algorithm software) embedded in a flexible manner. In the form of a flexible belt, controller 520 is portable and well suited for comfortable wearing by a patient on a daily basis and may be shifted around the waist of the subject (or worn elsewhere) for comfort. As shown in FIG. 5B, controller belt 520 comprises a belt 528 comprising a flexible material and compartments 526 for housing of the components of defibrillation controller 520. It should be appreciated that distributing the components of defibrillation controller 520 between compartments 526 provides the required flexibility and overcomes the discomfort of wearing or carrying a box. It should further be appreciated that controller belt 520 does not need to touch the patient's body or skin and therefore is not a source of skin irritation. Only patches 510 need to be in contact with the patient's skin. It should further be appreciated that the controller is not attached to the subject via a patch touching the subject's skin.

Wiring (not shown) between compartments 526 provides for the electrical connections between the components of controller 520. In some embodiments, compartments 526 are cylindrical in shape. In some embodiments, compartments 526 have the form of a pocket. In some embodiments, compartments 526 are rectangular in shape. In some embodiments, belt 520 is a lightweight belt. In some embodiments, belt 520 including the patches 526 and cables 524 weighs less than 500g.

Cables 524 enables removal of controller belt 520 from the subject for placing proximal to the patient while patches 510 remain attached to the subject to enable, for example, sleeping. In some embodiments, cables 524 are extendable for placing controller belt 520 further away from the subject. In some embodiments, compartments 526 are attached to controller belt 520 using flexible spacers (not shown).

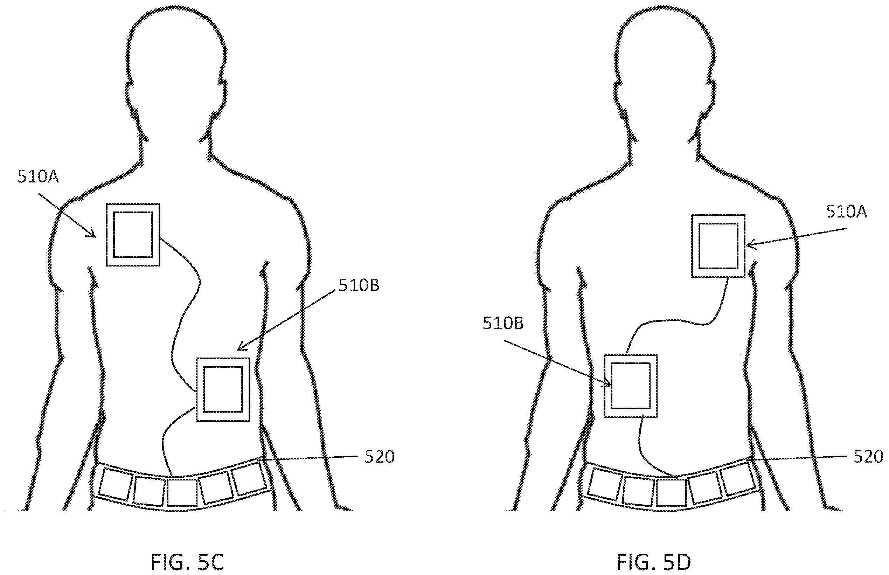

In some embodiments, replaceable patches 510A, 510B can be placed in alternate locations to eliminate skin damage which may occur due to long-term usage of the patch in one position. A method for overcoming skin irritations which may be caused by patches and adhesives is shown in FIGS. 5C- 5F. Defibrillators typically have one shocking electrode on the right side of the chest and a second shocking electrode on the left abdomen to provide a known shock vector. The inventors have determined that alternative shock vectors are possible, thus allowing periodic movement of the patches 510 to alternative positions to reduce long-term skin irritation.

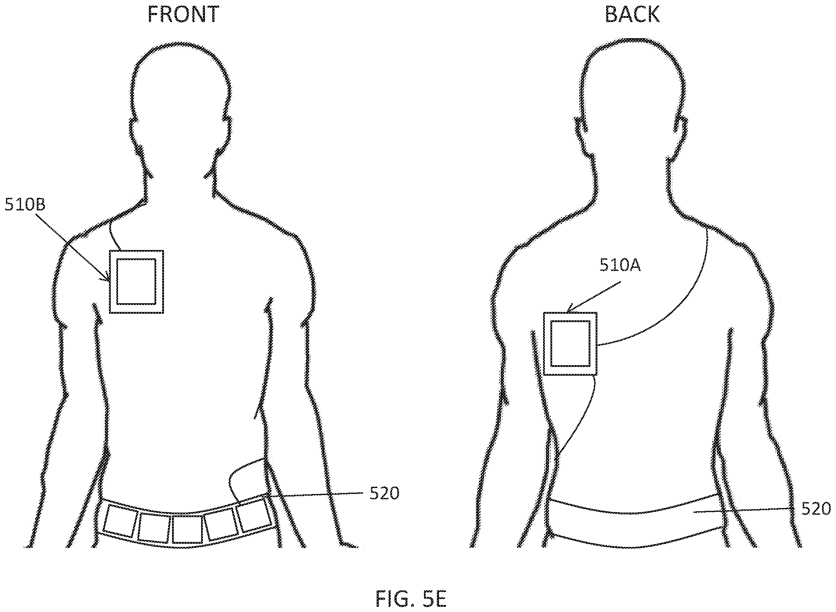

In FIG. 5C, patches 510A and 510B are shown as located on the right side of the chest and left abdomen respectively. When a patch position location change is desired (normally within a week to avoid skin irritation), a second shock vector is provided by positioning patch 510A to the left side of the chest while patch 510B is positioned to the right side of the abdomen as shown in FIG 5D. A third shock vector is provided by positioning patch 510B on the upper right side of the chest while patch 510A is positioned to the left upper side of the back as shown in FIG 5E.

Alternating the sides for patches 510 enables use of relatively large patches compared to standard AED patches (desirable for conductivity) while reducing potential discomfort of subjects.

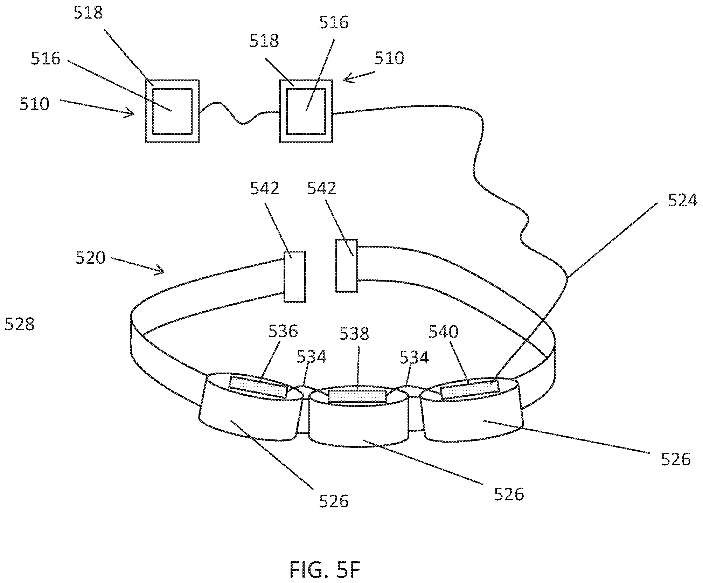

In the embodiment of FIG. 5F, controller 520 comprises batteries 536, a capacitor 538 and electronics 540. In the embodiment of FIG. 5F, wiring 534 provides for the electrical connections of the components of controller 520. It should be appreciated that the separation of the components of controller 520 into separate compartments 526 provides for greater comfort as belt 528 remains flexible and the weight is distributed around belt 528.

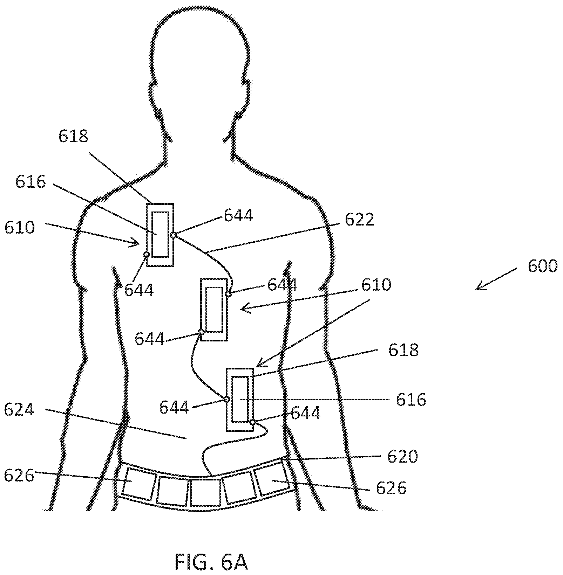

FIGS. 6A-6B show exemplary schematic drawings of a BCD according to some embodiments. As shown in FIG. 6A BCD 600 comprises replaceable patches 610 and defibrillation controller belt 620. Each of patches 610 comprises both of a defibrillation electrode 616 and an ECG sensor 618. Patches 610 are affixed to the body of the subject using biocompatible adhesive. Patches 610 are of smaller dimensions than patches typically used in the art. In some embodiments, patches 610 are smaller than 8x8cm. The smaller size enables changing of the patch location periodically to enhance patient tolerance to such patches.

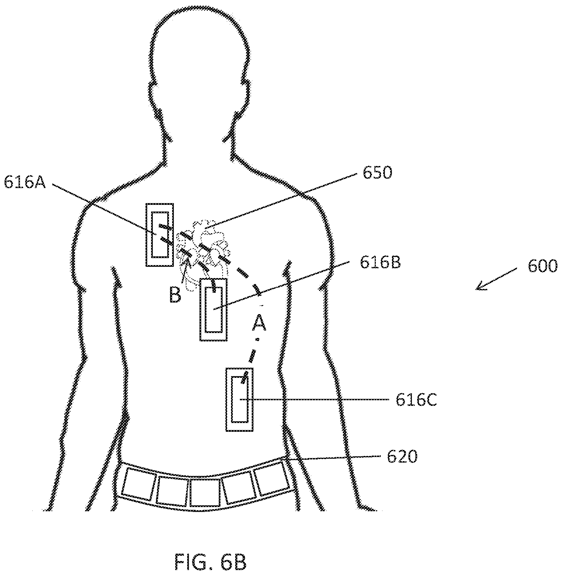

To enable sufficient energy to be conveyed by defibrillation electrodes 616 to the heart of a subject during a shock, more than two patches 610 are affixed to the subject. Multiple defibrillation electrodes 616 provide multiple shock vectors between the affixed defibrillation electrodes 616. As shown in FIG. 6B, a defibrillation vector "A" passes between electrode 616C through the heart 650 to electrode 616A, while defibrillation vector "B" passes between electrode 616B through heart 650 to electrode 616A. Although FIGS. 6A-6B show three patches 610, it should be appreciated that more than three patches may be used depending on the size of the patches and the amount of energy to be conveyed in a shock.

In some embodiments, patches 610 comprise gel for enhancing electrical contact. In some embodiments, replaceable patches 610 are replaced periodically. Cables 622 provide electrical connectivity between patches 610 via connectors 644 and cables 624 provide electrical connectivity between patches 610 (via connector 644) and controller belt 620 as described above with reference to FIGS. 5A - 5G. Controller belt 620 is the same as controller belt 520 as described above.

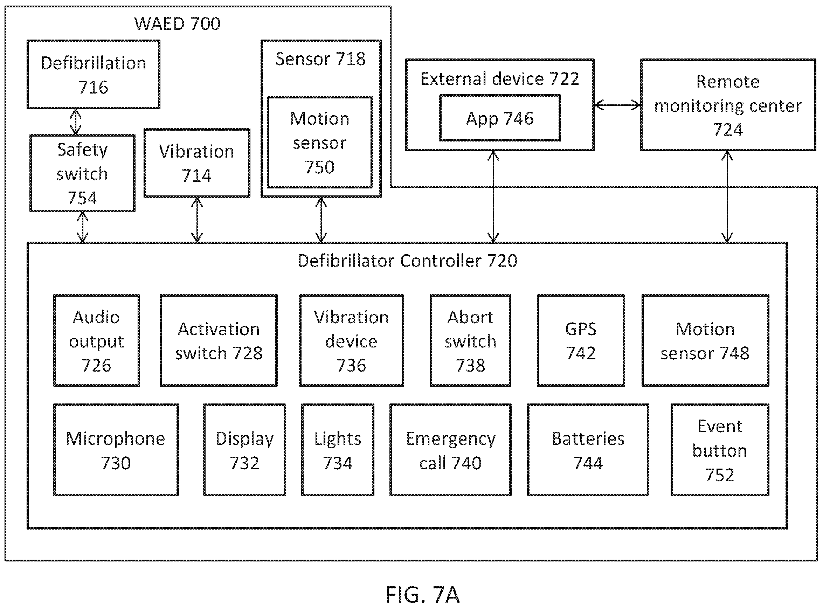

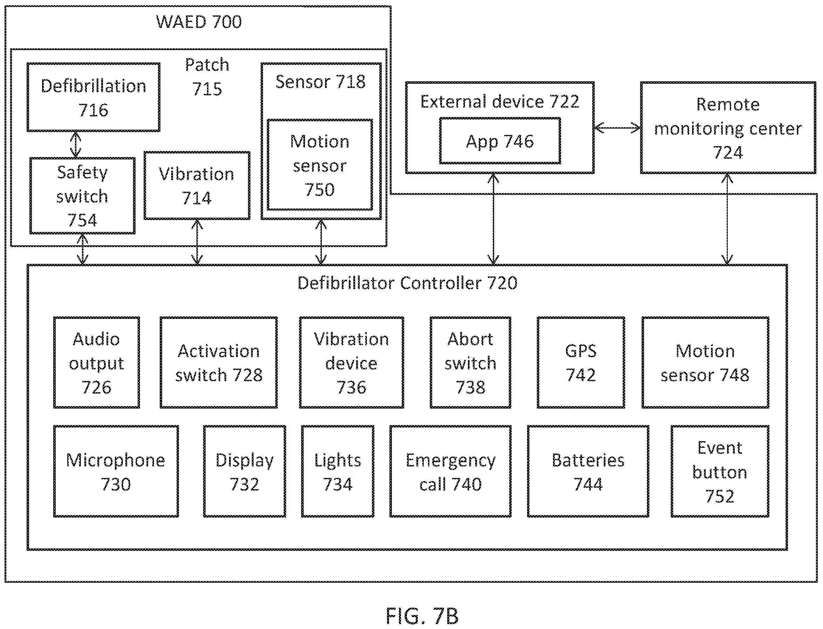

FIGS. 7A-7B show exemplary schematic drawings of the components of a BCD according to some embodiments. As shown in FIG. 7, BCD 700 comprises a defibrillation electrode 716, an ECG sensor 718 and a controller 720. In some embodiments, BCD 700 also comprises a vibration pad 714. One or more of these components are in communication with an external device 722 and/or a remote monitoring center (RMC) 724. Embodiments of controllers 120, 220, 320, 420, 520, and 620 are the same as controller 720. Embodiments of patches/electrodes 116, 118, 216, 218, 316, 318, 416, 418, 516, 518, 616, and 618 have the same characteristics as defibrillation electrodes 716 and sensors 718. In some embodiments, such as shown in FIG. 7B, electrodes 716 and sensors 718 are combined into a single patch 715 as with patches 510 or 610. In some embodiments, vibration patch 714 is integrated into one or both of patches 716 and 718.

It should be appreciated that the components of controller 720 as described below may be provided in a single housing (such as but not limited to the housings of controllers 120 or 420) or alternatively may be distributed into multiple housings such as but not limited to the embodiment of FIG. 5A-5B where controller 720 components are stored in compartments 526, or the embodiment of FIG. 3 where controller 720 is housed in housing 322.

In some embodiments, the ECG sensors 718 and controller are adapted for sensing simultaneous sensing vectors. The adaptation includes sensing from three or more sensing electrodes placed on two or more patches for better sensing and detection of VF and discrimination of non-VF arrhythmias.

In some embodiments, defibrillation electrodes 716 are adapted for providing multiple simultaneous shock vectors. The adaptation includes providing three or more shock electrodes each placed on a patch 715 for better coverage of the heart and reduction of defibrillation threshold energy.

In some embodiments, controller 720 comprises an activation switch 728 to turn BCD 700 ON once the patches are in place. In some embodiments, controller 720 comprises an audio output device 726 to generate an alarm sound such as beeping or buzzing to alert the patient in case VT or VF was detected and a shock is about to be delivered. Additionally, audio device 726 may play audio messages from RMC 724, or messages related to the BCD 700 status. In some embodiments, controller 720 also comprises a microphone 730 for hearing comments from the subject. In some embodiments, audio output device 726 sounds an audible alarm following administering a defibrillating shock to warn/notify passers-by.

In some embodiments, ECG data as sensed by sensor patch 718 is collected for storage by controller 720, analysis by controller 720 and/or transmission to RMC 724 for analysis and storage. In some embodiments, controller 720 includes an ECG event button 752 for creating a time stamp in the collected ECG data such as but not limited to when a patient feels an arrythmia or other sensation the patient wishes to record. In some embodiments, ECG event button 752 initiates recording via microphone 730 such that the patient can state the reason for pressing ECG event button 752. In some embodiments, ECG data is continually buffered but only stored/recorded by controller 720 when ECG event button 752 is pressed including recording a time period before ECG event button 752 was pressed.

In some embodiments, controller 720 comprises visual indication means such as a display 732 and/or status lights 734 for displaying the status of BCD 700 and/or displaying messages related to BCD 700 and use thereof. In some embodiments, controller 720 measures impedance of patches 716, 718 to the body of the subject to determine the strength of the electrical connection therebetween. In some embodiments, controller 720 displays the measured strength of the electrical connection of a patch 716 and/or 718 to the skin of the subject. In some embodiments, controller 720 indicates the level of connection of patches 716 and/or 718 with the skin of the subject. In some embodiments, controller 720 indicates a suggested period for replacement or movement of patches 716, 718.

In some embodiments, controller 720 comprises a vibration device 736 to generate vibration to alert the patient in case VF was detected and a shock is about to be delivered. Additionally or alternatively, in some embodiments, a vibration alert is delivered to the subject via vibration patch 714.

In some embodiments, controller 720 comprises an abort button 738 used to abort a shock in a case the patient feels fine but the BCD 700 is alerting that a shock is about to be delivered. In some embodiments, controller 720 comprises an emergency call button 740. In some embodiments, safety switch 754 electrically disconnects controller 720 from defibrillation patch 716. In some embodiments, safety switch 754 is activated by abort button 738.

In some embodiments, controller 720 is in wireless communications with an RMC 724. In some embodiments, controller 720 notifies RMC 724 of an administered defibrillation shock. In some embodiments, controller 720 continuously analyzes the signal from ECG patch 718 to determine whether an arrhythmia has occurred. In some embodiments, detected arrhythmia events are stored by controller 720. In some embodiments, detected arrhythmia events are transmitted to RMC 724. In some embodiments, controller 720 comprises a GPS receiver 742, and the location of controller 720 is transmitted along with any information transmitted to RMC 724. In some embodiments, a patient can initiate a voice call with medical staff at the RMC 724 using microphone 730 and audio output 726.

In some embodiments, controller 720 includes a motion sensor 748. Motion sensor 748 may include one or more of a gravity sensor, linear acceleration sensor, rotation vector sensor, step counter, step detector, accelerometer and/or gyroscope. In some embodiments, sensor patch 718 includes a motion sensor 750. Motion sensor 750 may include one or more of a gravity sensor, linear acceleration sensor, rotation vector sensor, step counter, step detector, accelerometer and/or gyroscope. In some embodiments, one or both of motion sensors 748, and 750 may determine data related to movement of the patient. In some embodiments, movement data is collected by controller and combined with the ECG data provided sensor patch 718. In some embodiments, motion sensor data, indicative of the movement of the patient, is used as part of monitoring of syncope by controller 720 and/or by RMC 724.

In some embodiments, controller 720 comprises batteries 744 that can be replaceable and/or rechargeable. In some embodiments, rechargeable batteries 744 use either wireless charging or are charged using a cable (not shown) connected to the controllers from a power source.

In some embodiments, controller 720 is in wireless communication with external device 722. Non-limiting examples of external device 722 include a smartphone or smart watch. External device 722 comprises a software application (app) 746 for running on the external device. In some embodiments, app 746 presents the status of BCD 700 using the display of the external device such as, for example, to indicate a need for charging of BCD 700 or to indicate an error in the functioning of BCD 700. In some embodiments, the audible, visual and/or vibrating indication of an impending shock is provided by app 746 using the display, audio, and/or vibrating functions of external device 722. In some embodiments, an abort button is provided by app 746 and the touchscreen capability for activating the abort button is provided by external device 722. In some embodiments, an indication of the impending shock or activation of a shock is transmitted from controller 720 to external device 722 for further transmission by external device 722 using the built-in data communication functionality of external device 722 to a RMC 724, such as for emergency response. In some embodiments, app 746 provides BCD 700 status information. In some embodiments, app 746 provides guided instructions for patch placement and rotation. In some embodiments, data from ECG patch 718 is provided to app 746 for analyzing of the signal from ECG patch 718 to determine whether an arrhythmia has occurred. In some embodiments, detected arrhythmia events are stored by app 746 on device 722. In some embodiments, detected arrhythmia events are transmitted by app 746 to RMC 724 using the communication features of device 722. In some embodiments, event button 752 is activated via app 746.

In some embodiments, when BCD 700 detects asystole after delivering a shock, BCD 700 delivers high voltage temporary external percutaneous heart pacing via patches 716. In some embodiments, the pacing is ventricular demand pacing (VVI). In some embodiments, the pacing is provided at a rate of 35 BPM.

In some embodiments, each patch 716, 718 comprises multiple perforations throughout the patch allowing for breathing of the skin while the patch is applied.

In the claims or specification of the present application, unless otherwise stated, adjectives such as "substantially" and "about" modifying a condition or relationship characteristic of a feature or features of an embodiment, are understood to mean that the condition or characteristic is defined to within tolerances that are acceptable for operation of the embodiment for an application for which it is intended.

It should be understood that where the claims or specification refer to "a" or "an" element, such reference is not to be construed as there being only one of that element.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

While this disclosure describes a limited number of embodiments, it will be appreciated that many variations, modifications and other applications of such embodiments may be made. The disclosure is to be understood as not limited by the specific embodiments described herein, but only by the scope of the appended claims.

## Claims

1. A flexible automated wearable belt cardiac defibrillator, BCD, (500, 600, 700) for wearing by a subject, the subject having a body, the BCD **characterized by** comprising:
at least two disposable patches (510, 610, 710) adapted for adhering to the body of the subject, each patch comprising a defibrillation electrode (516, 616, 716), wherein at least one patch includes an ECG sensor (518, 618, 718); and
a controller (520, 620, 720) that is separate from the patches and connected via a connection cable (524, 624) to each patch,
wherein the patches comprise an adhesive adapted for long-term adhering of the patches to the body of the subject,
wherein the patches and the adhesive are adapted for movement of the subject while the patches are adhered to the body of the subject,
wherein the controller is housed in a flexible belt (528) comprising a plurality of compartments (526, 626) for wearing by the subject, is adapted for being flexible, and is not required to be in contact with the body of the subject,
and wherein the adaptation for being flexible comprises distributing components of the controller between the plurality of compartments.

2. The BCD of claim 1, wherein the controller components comprise a battery (744), at least one high voltage and high energy capacitor (538), and controller electronics (540), and wherein each of the controller components is housed in a separate one of the plurality of compartments.

3. The BCD of any of the claims 1 or 2, wherein at least one of the patches comprises a vibration element (530).

4. The BCD of any of the preceding claims, wherein the at least two patches include at least three patches for providing at least two defibrillation vectors.

5. The BCD of any of the preceding claims, wherein the controller is adapted for storage and/or analysis of ECG sensor data sensed by the at least one ECG sensor.

6. The BCD of claim 5, wherein the controller comprises an ECG event button (752) adapted to be pressed to create a time stamp in the collected ECG sensor data.

7. The BCD of any of the preceding claims, wherein the controller is adapted to measure impedance between each patch and a body of the subject to determine strength of an electrical connection therebetween.

8. The BCD of any of the preceding claims, wherein the controller comprises an abort button (738) adapted for aborting an impending shock from the defibrillation electrode.

9. The BCD of any of the preceding claims, wherein the patches comprise connectors for connecting or disconnecting interconnection cables between the patches and/or between the patches and the controller.

10. The BCD of any of the preceding claims, wherein the controller is adapted for data communication with a remote monitoring center, RMC (724).

11. The BCD of claim 10, wherein ECG data sensed by the ECG sensor is transmitted to the RMC for analysis and storage.

12. The BCD of claim 10, wherein detected arrhythmia events are transmitted to the RMC.

13. The BCD of claim 10, wherein the controller comprises a GPS receiver (742) adapted for determining the geolocation of the BCD, and wherein the determined location is transmitted to the RMC.

14. The BCD of claim 10, wherein the controller comprises a microphone (730) and an audio device (726) and is adapted for initiating a voice call with the RMC.

15. The BCD of any of the preceding claims, wherein the controller is adapted for wireless communication with an external device.

## Patentansprüche

1. Ein flexibler, automatisierter tragbarer Gürtel-Herzdefibrillator, GHD, (500, 600, 700) zum Tragen durch ein Subjekt mit einem Körper, wobei der GHD **dadurch gekennzeichnet ist, dass** er Folgendes umfasst:
wenigstens zwei Einwegpflaster (510, 610, 710), die zur Haftung an den Körper des Subjekts geeignet sind, wobei jedes Pflaster eine Defibrillatorelektrode (516, 616, 716) umfasst, wobei wenigstens ein Pflaster einen EEG-Sensor (518, 618, 718) umfasst; und
eine Steuerung (520, 620, 720), die von den Pflastern getrennt vorliegt und mittels eines Verbindungskabels (524, 624) mit jedem Pflaster verbunden ist,
wobei die Pflaster einen Klebstoff umfassen, der zur langfristigen Haftung der Pflaster an den Körper des Subjekts geeignet ist,
wobei die Pflaster und der Klebstoff für Bewegungen des Subjekts während der Anhaftung der Pflaster an den Körper des Subjekts geeignet sind,
wobei die Steuerung in einem flexiblen Gürtel (528) zum Tragen durch das Subjekt untergebracht ist, der eine Vielzahl von Kompartimenten (526, 626, 726) umfasst,
der flexibel ist und nicht notwendigerweise mit dem Körper des Subjekts in Kontakt steht,
und wobei die Eignung zur Flexibilität die Verteilung der Komponenten der Steuerung auf die Vielzahl der Kompartimente umfasst.

2. Der GHD von Anspruch 1, wobei die Steuerungskomponenten eine Batterie (744), wenigstens einen Hochspannungs- und Hochenergie-Kapazitator (538), und Steuerungselektronik (540) umfassen, und wobei jede der Steuerungskomponenten in einer der Vielzahl der Kompartimente getrennt untergebracht ist.

3. Der GHD von einem der Ansprüche 1 oder 2, wobei wenigstens eines der Pflaster ein Vibrationselement (530) umfasst.

4. Der GHD von einem der vorangegangenen Ansprüche, wobei die wenigstens zwei Pflaster wenigstens drei Pflaster zur Bereitstellung von wenigstens zwei Defibrillationsvektoren umfassen.

5. Der GHD von einem der vorangegangenen Ansprüche, wobei die Steuerung zur Speicherung und/oder Analyse der Daten des EEG-Sensors geeignet ist, die durch den wenigstens einen EEG-Sensor erkannt werden.

6. Der GHD von Anspruch 5, wobei die Steuerung wenigstens eine EEG-Ereignistaste (752) umfasst, die zum Drücken geeignet ist, um einen Zeitpunkt in den gesammelten EEG-Daten zu erzeugen.

7. Der GHD von einem der vorangegangenen Ansprüche, wobei die Steuerung zur Messung der Impedanz zwischen jedem Pflaster und einem Körper des Subjekts geeignet ist, um die Stärke einer elektrischen Verbindung dazwischen zu bestimmen.

8. Der GHD von einem der vorangegangenen Ansprüche, wobei die Steuerung eine Abbruchtaste (738) umfasst, die zum Abbruch eines bevorstehenden Shocks der Defibrillationselektrode geeignet ist.

9. Der GHD von einem der vorangegangenen Ansprüche, wobei die Pflaster Verbinder zur Verbindung oder Trennung der Verbindungskabel zwischen den Pflastern und/oder zwischen den Pflastern und der Steuerung umfassen.

10. Der GHD von einem der vorangegangenen Ansprüche, wobei die Steuerung zur Datenkommunikation mit einem Remote-Beobachtungszentrum (RBZ, 724) geeignet ist.

11. Der GHD von Anspruch 10, wobei die durch den EEG-Sensor aufgenommenen EEG-Daten an ein RBZ zur Analyse und Speicherung übermittelt werden.

12. Der GHD von Anspruch 10, wobei die nachgewiesenen Arrhythmieereignisse an das RBZ übermittelt werden.

13. Der GHD von Anspruch 10, wobei die Steuerung einen GPS-Empfänger (742) umfasst, der zur Bestimmung der Geoposition des GHDs geeignet ist, und wobei die bestimmte Position an das RBZ übermittelt wird.

14. Der GHD von Anspruch 10, wobei die Steuerung ein Mikrofon (730) und eine Audiovorrichtung (726) umfasst und zum Auslösen einen Sprachanrufs an das RBZ geeignet ist.

15. Der GHD von einem der vorangegangenen Ansprüche, wobei die Steuerung zur drahtlosen Kommunikation mit einer externen Vorrichtung geeignet ist.

## Revendications

1. Défibrillateur cardiaque se portant comme une ceinture (BCD) (500, 600, 700) flexible et automatisé destiné à être porté par un sujet, le sujet possédant un corps, le BCD étant **caractérisé en ce qu'**il comprend :
au moins deux patchs jetables (510, 610, 710) adaptés pour adhérer au corps du sujet, chaque patch comprenant une électrode de défibrillation (516, 616, 716), au moins un patch incluant un capteur d'ECG (518, 618, 718), et
un dispositif de commande (520, 620, 720) distinct des patchs et relié par un câble de liaison (524, 624) à chaque patch ;
les patchs comprenant un adhésif adapté pour une adhérence à long terme des patchs au corps du sujet,
les patchs et l'adhésif étant adaptés au mouvement du sujet tandis que les patchs adhèrent au corps du sujet,
le dispositif de commande étant logé dans une ceinture flexible (528) destinée à être portée par le sujet et comprenant une pluralité de compartiments (526, 626), étant adapté pour être flexible et n'ayant pas besoin d'être en contact avec le corps du sujet,
et l'adaptation pour être flexible comprenant la distribution de composants du dispositif de commande entre la pluralité de compartiments.

2. BCD selon la revendication 1, dans lequel les composants du dispositif de commande comprennent une batterie (744), au moins un condensateur haute tension et haute énergie (538) et une électronique de dispositif de commande (540), et dans lequel chacun des composants du dispositif de commande est logé dans un compartiment séparé de la pluralité de compartiments.

3. BCD selon l'une quelconque des revendications 1 ou 2, dans lequel au moins l'un des patchs comprend un élément vibratoire (530).

4. BCD selon l'une quelconque des revendications précédentes, dans lequel les au moins deux patchs incluent au moins trois patchs permettant de fournir au moins deux vecteurs de défibrillation.

5. BCD selon l'une quelconque des revendications précédentes, dans lequel le dispositif de commande est adapté au stockage et/ou à l'analyse de données de capteur d'ECG détectées par l'au moins un capteur d'ECG.

6. BCD selon la revendication 5, dans lequel le dispositif de commande comprend un bouton d'événement d'ECG (752) adapté pour être pressé pour créer un horodatage dans les données de capteur d'ECG collectées.

7. BCD selon l'une quelconque des revendications précédentes, dans lequel le dispositif de commande est adapté pour mesurer l'impédance entre chaque patch et le corps du sujet pour déterminer l'intensité d'une liaison électrique entre eux.

8. BCD selon l'une quelconque des revendications précédentes, dans lequel le dispositif de commande comprend un bouton d'interruption (738) adapté pour interrompre un choc imminent provenant de l'électrode de défibrillation.

9. BCD selon l'une quelconque des revendications précédentes, dans lequel les patchs comprennent des connecteurs pour relier ou séparer des câbles d'interconnexion entre les patchs et/ou entre les patchs et le dispositif de commande.

10. BCD selon l'une quelconque des revendications précédentes, dans lequel le dispositif de commande est adapté pour une communication de données avec un centre de télésurveillance (RMC).

11. BCD selon la revendication 10, dans lequel des données d'ECG détectées par le capteur d'ECG sont transmises au RMC pour analyse et stockage.

12. BCD selon la revendication 10, dans lequel tout événement d'arythmie détecté est transmis au RMC.

13. BCD selon la revendication 10, dans lequel le dispositif de commande comprend un récepteur GPS (742) adapté pour déterminer l'emplacement géographique du BCD, et dans lequel l'emplacement déterminé est transmis au RMC.

14. BCD selon la revendication 10, dans lequel le dispositif de commande comprend un microphone (730) et un dispositif audio (726) et est adapté pour lancer un échange vocal avec le RMC.

15. BCD selon l'une quelconque des revendications précédentes, dans lequel le dispositif de commande est adapté pour une communication sans fil avec un appareil externe.
